# EUROPÄISCHE PATENTSCHRIFT

(11) **EP 1 745 072 B1**
(45) Veröffentlichungstag und Bekanntmachung des Hinweises auf die Patenterteilung: **24.08.2011**
(21) Anmeldenummer: 05735130.6
(22) Anmeldetag: 16.04.2005
(51) Int. Cl.: C07K 14/705, C12N 15/63, G01N 33/569, G01N 33/50, G01N 33/68

(54) **VERFAHREN ZUR HERSTELLUNG LOESLICHER MHC-PROTEINE**
METHOD FOR PRODUCING SOLUBLE MHC PROTEINS
PROCEDE DE PRODUCTION DE PROTEINES MHC SOLUBLES

(30) Priorität: 06.05.2004 DE 102004022435
(43) Veröffentlichungstag der Anmeldung: 24.01.2007
(73) Patentinhaber: Imusyn GmbH Co. KG, 30625 Hannover (DE)
(72) Erfinder: BLASCZYK, Rainer, 30938 Burgwedel (DE)
(74) Vertreter: Kloiber, Thomas
(86) Internationale Anmeldenummer: PCT/EP2005/004056
(87) Internationale Veröffentlichungsnummer: WO 2005/108427

(56) Entgegenhaltungen:
- WO-A-93/10220
- US-A1- 2002 197 672
- SATO Y ET AL: "Improved generation of HLA class I/peptide tetramers" JOURNAL OF IMMUNOLOGICAL METHODS, ELSEVIER, AMSTERDAM, NL, Bd. 271, Nr. 1-2, 20. Dezember 2002 (2002-12-20), Seiten 177-184, XP004393936 ISSN: 0022-1759
- KAWANA-TACHIKAWA AI ET AL: "An efficient and versatile mammalian viral vector system for major histocompatibility complex class I/peptide complexes." JOURNAL OF VIROLOGY, Bd. 76, Nr. 23, Dezember 2002 (2002-12), Seiten 11982-11988, XP002355241 ISSN: 0022-538X
- FERNANDEZ-FRESNEDO GEMA ET AL: "Relationship of donor-specific class-I anti-HLA antibodies detected by ELISA after kidney transplantation on the development of acute rejection and graft survival." NEPHROLOGY DIALYSIS TRANSPLANTATION, Bd. 18, Nr. 5, Mai 2003 (2003-05), Seiten 990-995, XP002355242 ISSN: 0931-0509

## Beschreibung

Die Erfindung betrifft ein rekombinantes, aufgereinigtes MHC-Protein, welches im wesentlichen die gleiche Konformation, funktionelle Aktivität sowie die Bindungseigenschaften für spezifische Antikörper, Peptide, T-Zellrezeptoren und NK-Zellen und Antigene wie das native MHC-Protein aufweist.

Humane Leukozyten Antigene, oder abgekürzt HLA Moleküle sind innerhalb der adaptiven Immunabwehr von zentraler Bedeutung. Ihre Funktion besteht darin, an der Zelloberfläche Peptide gegenüber T-Lymphozyten zu präsentieren. Die Antigenspezifität der T-Zellen hängt dabei vom Major Histokompatibility Complex, abgekürzt MHC, Genotyp ab, was als MHC Restriktion bezeichnet wird. Diese MHC-gebundene Spezifität stellt die Basis für die Fähigkeit des Immunsystems dar, zwischen Selbst und Nicht-Selbst, z.B. Infektionserregern, viral infizierten Zellen oder Allotransplantaten, zu unterscheiden. Die Gene des HLA Komplexes sind auf dem kurzen Arm von Chromosom 6, 6p21, lokalisiert. Aus historischen Gründen werden drei MHC Regionen unterschieden, die zentromerwärts als Klasse II oder Klasse III sowie telomerwärts als Klasse I bezeichnet werden. Insbesondere HLA Klassen I und II sind durch einen enormen allelischen Polymorphismus gekennzeichnet, welcher nach jetzigem Kenntnisstand unter anderem 540 HLA-B Allele, und 418 HLA-DRB Allele umfasst. Bei HLA Klasse I Molekülen handelt es sich um heterodimere Glykolproteine, welche aus einer schweren, variablen α-Kette und einer leichten, invariablen β-Kette, β2-Mikroglobulin, zusammengesetzt sind. Teile der α1 und α2 Domänen bilden zusammen die Peptid-bindende Region PBR. In dieser grubenartigen Vertiefung der Plattformstruktur werden okta- bis undecamere Fragmente aus endogenen Proteinen nichtkovalent gebunden, und gegenüber CD8⁺ zytotoxischen T-Zellen präsentiert. Die α1 und α2 Domänen sind besondere im Bereich der PBR durch einen ausgeprägten Aminosäure Polymorphismus gekennzeichnet, wohingegen die Variabilität in den weiteren Molekülabschnitten wesentlich geringer ist.

Auch bei HLA Klasse II handelt es sich um heterodimere Moleküle. Diese bestehen aus jeweils einer αund einer β Kette, welche sich im Molekulargewicht geringfügig voneinander unterscheiden. Bei HLA Klasse II sind beide Ketten mit ihrer α1 bzw. β1 Domäne gemeinsam an der Bildung der Plattformstruktur beteiligt. Im Gegensatz zu HLA Klasse I werden hier über den exogenen Antigenpräsentationsweg aufgenommene Proteinfragmente mit einer Länge von 15-24 AS präsentiert. HLA Klasse II Moleküle werden auf professionell antigenpräsentierenden Zellen (Makrophagen, dendritische Zellen, B-Lymphozyten) sowie auf aktivierten T-Zellen exprimiert. Die Präsentation der Peptide erfolgt gegenüber CD4⁺ inflammatorischen T-Zellen oder T-Helferzellen. Die bei HLA Klasse I Molekülen an den α1 und α2 Domänen, und bei HLA Klasse II in den α1 und β1 Domänen vorhandenen Aminosäure Polymorphismen legen das Peptidbindungsverhalten des jeweiligen HLA Moleküls sowie die Konformation der präsentierten Peptide fest.

Aufgrund der in den industrialisierten Ländern bestehenden geringen durchschnittlichen Familiengröße kann nur bei ca. 30 % der für eine Stammzellentransplantation in Frage kommenden Patienten ein HLA genotypisch identischer Geschwisterspender zur Verfügung gestellt werden. Aus diesem Grunde werden mit steigender Häufigkeit Blutstammzellen von HLA gematchten Fremdspendern transplantiert. Ein wesentliches Problem bei der allogenen Blutstammzellentransplantation stellt die Graft-versus-host Erkrankung (GVHD) dar, bei welcher Donor-Lymphozyten allogene HLA Merkmale im Empfängergewebe erkennen. Der Einfluss von HLA Mismatchen auf das Risiko der Entwicklung einer schweren akuten GVHD oder einer Transplantatabstoßung (Host-versus-graft Reaktion) wurde in einer Reihe von Studien untersucht. In der überwiegenden Mehrzahl der Untersuchungen stellten sich HLA Klasse I Mismatche, insbesondere für HLA-A und B, als deutliche Risikofaktoren sowohl in GVH, als auch in der Host-versus-graft (HVG) Richtung heraus. Hinsichtlich HLA Klasse II waren bei der überwiegenden Mehrzahl der Studien insbesondere Mismatche an den Genorten, welche DRB1 und DQB1, welche die β-Ketten der HLA-DR und DQ Merkmale codieren, mit einem erhöhten Risiko für die Entstehung einer schweren akuten GVHD verbunden. Die Bedeutung von Mismatchen am DPB1 Genort wird derzeit kontrovers diskutiert. Hinsichtlich der übrigen HLA Klassen II Genorte liegen derzeit für eine Bewertung deren Transplantationsrelevanz zuwenig Daten vor. Unter Berücksichtigung der gegenwärtigen Datenlage besteht in Deutschland derzeit der Konsensus, dass bei der allogenen Blutstammzellentransplantation des HLA Matching von Donor und Rezipient die HLA Klasse I Merkmale HLA-A und B, d.h. niedrigauflösende Typisierung auf Allelgruppenebene, sowie die Klasse II Genorte DRB1 und DQB1, d.h. hochauflösende molekulargenetische Untersuchung auf Allelebene umfassen soll. Am Stichtag 22. Juli 2003 standen weltweit 8.575.256 potenzielle Stammzellspender bzw. Nabelschnurbluteinheiten zur Verfügung, wobei diese Zahl aufgrund von Informationskampagnen und Aufrufen zur Stammzellspende kontinuierlich wächst. Somit kann davon ausgegangen werden, dass zukünftig für eine wachsende Zahl von Patenten mehrere phänotypisch HLA identische Fremdspender verfügbar sein werden, und die Anforderungen an die Spender-Empfängerauswahl z. B. durch Erhöhung der Untersuchungsgenauigkeit oder durch Einbeziehung weiterer Merkmale erhöht werden können.

Die Organtransplantation stellt heute ein standardisiertes Verfahren zur Behandlung von Organversagen dar. Die am häufigsten transplantierten Organe betreffen Herz, Leber, Lunge, Niere und Pankreas. Im Jahr 2003 wurden im Eurotransplantverbund Belgien, Deutschland, Luxemburg, Niederlande, Österreich, Slovenien ca. 3.500 Nieren, 1.100 Lebern, 400 Pankreas, 600 Herzen und 300 Lungen transplantiert.

Nach gegenwärtigem wissenschaftlichen Kenntnisstand ist die Gewebeverträglichkeit zwischen Spender und Empfänger in der Übereinstimmung der Antigene des HLA-Systems begründet. Für Nierenund Herztransplantationen werden die besten Ergebnisse bei völliger Identität des HLA-Antigene zwischen Spender und Empfänger erzielt. Es besteht kein Zweifel, dass immunologische Abstoßreaktionen gegen diese Organe in ihrer Inzidenz und Stärke deutlich vermindert und therapeutisch besser beherrschbar sind, wenn eine höchstmögliche Übereinstimmung für die HLA-Antigene besteht. Für die Transplantation von Leber, Lunge und Pankreas konnte klinisch bisher nicht nachgewiesen werden, dass durch HLA-Übereinstimmungen zwischen Patienten und Spender das immunologische Abstoßungsrisiko vermindert werden kann. Es konnte jedoch gezeigt werden, dass präformierte oder nach Transplantationen gebildete spenderspezifische anti-HLA-Antikörper in jedem Fall zu einer humoralen Abstoßung bis zum Funktionsverlust des transplantierten Organs führen.

Die Detektion von anti-HLA Antikörpern ist im Rahmen der Transplantation von Blutstammzellen und soliden Organen ein zentraler Bestandteil der Prä- und Posttransplantations-Diagnostik. Bei allen Patienten zur Stammzellentransplantation und allen Patienten, die auf den Wartelisten zur Transplantation solider Organe geführt werden, erfolgt immer vor Transplantation und in Abhängigkeit vom klinischen Verlauf nach Transplantation ein Screening nach HLA-Antikörpern. Bei Patienten zur Nierentransplantation wird dieses Screening gemäß den Richtlinien der Eurotransplant Foundation sogar quartalsweise durchgeführt, um HLA-Antikörper-positive Patienten auf den Wartelisten sicher zu identifizieren. In ca. 20 % der Patienten werden HLA-Antikörper gefunden, die zur Charakterisierung nicht transplantabler HLA Antigene in weiteren Untersuchungen spezifiziert werden müssen. Die zum Screening und zur Spezifizierung verwendeten Methoden basieren auf Zytotoxizitätstechniken mit vitalen Lymphozyten oder ELISA Techniken mit aus Zelllinien gewonnenen natürlichen HLA Molekülen. Aufgrund der Co-Expression aller HLA Loci sowie dem Kopplungsungleichgewicht zwischen Allelen der verschiedenen HLA Loci ist eine Identifizierung der Antikörperspezifitäten dadurch nur sehr eingeschränkt möglich. Bei hochimmunisierten Patienten ist eine Spezifierung der Antikörper mit diesen Techniken in fast keinem Fall möglich. Aber gerade in diesem Fällen ist die Identifizierung akzeptabler Mismatche, d. h., von HLA Antigenen, gegen die keine Antikörper gebildet worden sind, besonders bedeutsam, da dies häufig die einzige Chance bietet, einen kompatiblen Spender zu ermitteln.

Durch die Verwendung rekombinanter humaner MHC-Proteine, rhMHC, können diese Restriktionen durch Verwendung eines definierten Antigens pro Reaktion als ELISA oder durch durchflusszytometrische Technik gelöst werden. Durch Verwendung aller rhMHC Antigene pro Reaktion kann zudem ein effektives Antikörper-Screening System etabliert werden. Mit rhMHC kann weiterhin die Problematik der begrenzten Verfügbarkeit seltener HLA-Allele für Screeningzwecke gelöst werden. Rekombinante humane MHC stehen seit kurzem als prokaryote und eukaryote Proteine zur Verfügung, die nach den unten beschriebenen Verfahren hergestellt werden können.

Das langfristige Ziel einer allogenen Blutstammzellentransplantation SCT ist zum einen die Etablierung einer dauerhaften T-Zellantwort gegen MHC restringierte Tumorantigene und zum anderen die Entstehung einer Toleranz gegenüber gesunden Zellen. Das Potential allogener T-Zellen für die Kontrolle von malignem Zellwachstum ist aus der engen Kopplung zwischen Graft versus Host GvH und Graft versus Leukemia (GvL) Reaktion bekannt und konnte eindrucksvoll durch die Reinduktion von Remissionen durch T-Zellen nach Stammzelltransplantationen gezeigt werden. Unter Berücksichtigung der bislang enttäuschenden Ergebnisse der autologen Immuntherapie maligner Erkrankungen mit einerseits modifizierten Tumorzellen, Tumorlysaten oder Hybridzellen und andererseits Ansätzen zur spezifischen Peptid-, Protein- oder DNA-Vakzinierungen kann vermutet werden, dass eine erfolgreiche Immuntherapie eher im allogenen als im autologen System zu erwarten ist. In die gleiche Richtung weisen die bisherigen Daten zur Identifizierung tumorassoziierter T-Zellepitope. Der allogene Ansatz beinhaltet auf der anderen Seite den wesentlichen Nachteil der Abstoßung und der schweren GvH Reaktion, die einem generellen Einsatz von Stammzellen zur Zeit entgegenstehen. Um den Verlauf eines allogenen Ansatzes therapeutisch gezielter steuern zu können, ist die Charakterisierung von MHC Liganden sowie deren gewebespezifische bzw. tumorspezifische Präsentation ein unumgängliches Ziel. Dazu war der Abschluss des Humanen Genomprojektes ein wesentlicher Meilenstein, der es in zunehmenden Maße ermöglicht, komplette zellspezifische Expressionsprofile zu definieren und der die Basis für das Nachfolgeprojekt der genomweiten Identifizierung von Single Nucleotide Polymorphismen (SNP) liefert. Mit zunehmendem Voranschreiten des SNP Projektes ist unmittelbar eine stetige Identifizierung potentieller allogen relevanter MHC Liganden zu erwarten. Eine vergleichbare Entwicklung ist in der Infektionsbiologie mit fortschreitender Aufklärung mikrobieller Genome und MHC allelspezifischer Peptidbindungsmotive zu erwarten.

Virus-spezifische und die Mehrzahl alloreaktiver T-Zellen erkennen MHC Moleküle in Peptid-abhängiger Weise. Die identifizierung und Klonierung Virus- und Allopeptid-restringierter T-Lymphozyten ist mit konventionellen Methoden (Zytotoxizität oder Zytokineexpression) technisch sehr schwierig und mit einem sehr hohen Anteil unspezifischer Ergebnisse belastet. Mit Hilfe prokaryonter tetramerer HLA Komplexe konnten Peptid-selektive CD8⁺ T-Zellen nach viralen Infektionen sowie bei Autoimmunerkrankungen und Tumorpatienten direkt nachgewiesen werden. Neben der direkten Visualisierung konnte gezeigt werden, dass Tetramere zur Sortierung und Klonierung allorestringierter CTLs geeignet sind. Parallel mit der Identifizierung potentieller allogener und mikrobieller MHC Liganden wird der Bedarf nach rhMHC steigen, die mit diesen Liganden bestückt für die Identifizierung targetierbarer T-Zellepitope eingesetzt werden können. Sobald MHC allelspezifische T-Zellepitope identifiziert worden sind, kann die Kaskade diagnostischer und therapeutischer Applikationen einsetzen. Das gleiche gilt für die Detektion von NK-Zellen, deren Rezeptoren Liganden für MHC-Proteine darstellen. Hier ist jedoch noch unklar, ob diese Interaktion Peptid-selektiv oder unabhängig vom präsentierten Peptid erfolgt.

Rekombinante humane MHC-Proteine können in Prokaryonten oder Eukaryonten hergestellt werden. Die Herstellung in Eukaryonten erfolgt entweder trunkiert oder komplett. Die trunkierte Variante wird wie bei der prokaryonten Expression ohne den transmembranen Abschnitt und ohne den cytoplasmatischen Abschnitt exprimiert. Dadurch fehlt dem rekombinanten Molekül die Möglichkeit der Verankerung in der Zellmembran, so dass das Protein von den Zellen sezerniert wird. Ein solches Protein ist in den US-Offenlegungsschriften 2003/0191286 A1 und 2002/0197672 A1 beschrieben. Bei diesem Protein besteht der Nachteil, dass dem Protein der cytosolische Schwanz fehlt, obwohl er zum löslichen Teil des Proteins gehört.

Bei der Herstellung kompletter rekombinanter Moleküle wird das Protein in der Zellmembran exprimiert. Hierbei liegt der Nachteil aber darin, dass keine löslichen Proteine produziert werden.

Die Herstellung in Prokaryonten erfolgt als trunkiertes Molekül, wobei das Molekül ohne den transmembranen Abschnitt und ohne die cytoplasmatischen Abschnitte exprimiert wird. Dabei werden die einzelnen Ketten unabhängig in Prokaryonten exprimiert und in vitro zu einem funktionsfähigen MHC Molekül gefaltet. Neben dem Fehlen des cytosolischen Schwanzes bestehen die Nachteile dieser Methode darin, dass der Faltungsaufwand sehr hoch ist, den Proteinen die natürliche Glykosilierung fehlt und in der Regel nur ein einziges synthetisch hergestelltes Peptid, das zur Faltung verwendet wird, präsentiert wird.

Aufgabe der Erfindung ist es deshalb, ein MHC-Protein und ein Verfahren zu seiner Herstellung vorzuschlagen, welches löslich ist, aber dennoch die gleiche Faltung, Aktivität und die außerhalb der Membran für Antikörper zugänglichen Epitope aufweist, wie das native Protein.

Eine Lösung dieser Aufgabe sieht überraschenderweise vor, dass das rekombinante Protein löslich und nicht trunkiert, also am Ende abgeschnitten, ist. Hierdurch ergeben sich mehrere Vorteile. Durch die Löslichkeit ist zum einen die Ausbeute bei der Produktion der rekombinanten Proteine höher. Die Proteine verbleiben nicht in oder auf der Zellmembran, sondern werden sezerniert. Dadurch sind sie leichter zu ernten, weil sie nur noch aus dem Überstand des Zellkultivierungsmediums abgeschöpft werden brauchen. Zum anderen werden die produzierenden Zellen nicht beschädigt oder zerstört, was bei membranenständigen Proteinen nötig ist, um diese aus oder von der Membran zu entfernen. Auch hierdurch wird die Ausbeute verbessert. Durch ihre Löslichkeit sind die Proteine in Lösung stabiler und besser aufzubewahren. Da die meisten physiologischen Reaktionen in wässriger Lösung ablaufen, ist es von Vorteil, wenn auch die als Reagenz verwendeten Proteine löslich sind. Hierdurch sind sie vielseitig verwendbar. Weitere Vorteile des erfindungsgemäßen Proteins ergeben sich dadurch, dass es nicht trunkiert ist. Bei herkömmlichen löslichen MHC-Proteinen ist die Aminosäuresequenz ab der Position komplett abgeschnitten, die den Beginn der transmembranen Domäne darstellt. Die Trunkierung kann beispielsweise durch eine geeignete Protease vollzogen werden. Nach dem üblichen Verfahren geschieht dies dadurch, dass mittels PCR die Nucleotidsequenz nur bis zu dem Exon amplifiziert wird, welches vor dem den Membranteil kodierenden Exon liegt. Hierdurch wird dieses Exon zwar nicht amplifiziert, allerdings auch alle in der Sequenz folgenden Exons nicht, die die intrazelluläre Domäne des Proteins kodieren. Diese Domäne kann aber für die Funktion und Konformation wichtig sein. Außerdem ist sie Träger von Epitopen für gegen MHC gerichtete spezifische Antikörper. Daher ist es von erheblichem Vorteil, wenn ein rekombinantes MHC-Protein diese Domäne aufweist.

Die transmembrane Domäne des MHC-Proteins sorgt für dessen Verankerung in der Membran und verursacht die lipophilen Eigenschaften des Proteins, die eine Löslichkeit verhindern. Das MHC-Protein kann nun dadurch in ein lösliches Protein überführt werden, dass es keine transmembrane Domäne aufweist. Dies kann dadurch erreicht werden, dass beispielsweise beim Klasse 1 Allel mittels PCR und geeigneter Primer nur die Allelabschnitte bzw. Exons amplifiziert werden, welche für die hydrophilen Domänen, also α1, α2, α3 und den Schwanzteil kodieren, während das den transmembranen Teil kodierende Exon nicht amplifiziert wird. Wird die so amplifizierte ununterbrochene Basensequenz mittels geeigneter Methoden zur Expression des Proteins verwendet, dann bleibt der C-terminale Anteil des Proteins im wesentlichen unverändert. Das Protein weist also alle Domänen einschließlich des cytosolischen Schwanzes mit Ausnahme des transmembranen Teils auf. Eine andere Möglichkeit besteht darin, im Prinzip das gesamte Protein einschließlich des Membranteiles zu exprimieren, aber diesen hinsichtlich seiner Aminosäuresequenz zu modifizieren. Beispielweise können ein oder mehrere Codons innerhalb des den Membranteil kodierenden Exons verändert werden, so dass hydrophobe gegen hydrophile Aminosäuren ausgetauscht werden. Der Austausch eines Codons kann auch bezwecken, dass die Konformation der transmembranen Domäne funktionell derart verändert wird, dass sie ihre Verankerungsfunktion nicht mehr erfüllen kann und somit das gesamte Protein löslich wird. Hierzu kann bereits der Austausch eines Codons bzw. einer Aminosäure ausreichend sein.

Dadurch dass das rekombinante MHC-Protein einen cytosolischen Schwanz aufweist, hat es annähernd die gleiche Konformation, funktionelle Aktivität und Epitop-Struktur wie das native Protein.

Vorteilhaft ist es, dass das erfindungsgemäße rekombinante MHC-Protein die Glykolisierung des nativen MHC-Proteins aufweist. An Proteine gebundene Kohlenhydrate sind essentiell für die Konformation und Funktion dieser Proteine. Die Glykolisierung ist außerdem wichtig für die Erkennung von MHC-Proteinstrukturen durch Komponenten des Immunsystems, beispielsweise Antikörpern.

Das rekombinante MHC-Protein lässt sich dadurch isolieren, dass es mittels Affinitätschromatographie und/oder Gelchromatographie aufgereinigt wird. Eine einfache Methode zur affinitätschromatographischen Aufreinigung besteht in der Anwendung eines monoklonalen Antikörpers gegen die tag-Sequenzen der rekombinanten Moleküle z. B. antis-His, anti-V5.

Das rekombinante MHC-Protein kann ein HLA-Protein der Klasse I oder der Klasse II sein. Wie oben beschrieben wurde, werden rekombinante HLA-Proteine, also humane MHC-Proteine, dringend zum Screening von Transplantationspatienten benötigt.

Wenn das rekombinante MHC-Protein an seiner Peptid-bindenden Region ein dort gebundenes endogenes Peptid aufweist, hat dies eine Reihe von Vorteilen. Zum einen kann das MHC-Protein zum Nachweis des präsentierten Peptids durch Edman Sequenzierung und Massenspektrometrie des eluierten Peptides verwendet werden. Dadurch lassen sich zum einen Peptidbindungsmotive des betreffenden HLA Allels nachweisen und zum anderen die in der jeweiligen Zelle exprimierten Proteine, die dann als Peptid in dem rekombinanten HLA Molekül erscheinen, identifizieren. Zum anderen kann der Nachweis von Peptidbindungsmotiven für Vakzineentwicklungen bedeutsam sein. Außerdem kann der Nachweis von Peptiden aus endogen synthetisierten Proteinen der transfizierten oder transduzierten Zelle in Tumorzellen Hinweise auf relevante Tumorantigene liefern. Weiterhin kann der Nachweis von Peptiden aus endogen synthetisierten Proteinen der transfizierten oder transduzierten Zelle in natürlicherweise oder experimentell virusinfizierten Zellen Hinweise auf relevante Virusantigene liefern, die für Vakzineentwicklungen bedeutsam sein können.

Das beschriebene rekombinante MHC-Protein wird im wesentlichen nach folgendem Verfahren hergestellt:
a) Aufreinigung eines MHC Allels bestehend aus gDNA oder cDNA oder RNA.
b) PCR (Polymerase Chain Reaction) Amplifikation des MHC Allels von Exon 1 bis Exon 4 mit zwei geeigneten Primern, vorzugsweise mit dem Anfangs-Primer AE1 S und dem End-Primer AE4AS.
c) PCR Amplifikation eines MHC Allels, vorzugsweise des Allels des ersten oder zweiten Verfahrensschrittes, bis zum Ende der kodierenden Sequenz, die je nach MHC Allel in Exon 7 oder 8 liegt, also von Exon 6 bis Exon 7 oder Exon 8 mit zwei geeigneten Primern, vorzugsweise mit dem Anfangs-Primer AE6S_FS und dem End-Primer AE8AS_WOS, wobei der Anfangs-Primer, vorzugsweise AE6S_FS, eine 5' Sequenz enthält, die zum 3' Ende von Exon 4 komplementär ist, so dass über diese Sequenz eine Fusionssequenz hergestellt werden kann, und wobei der End-Primer, vorzugsweise AE8AS_WOS, kein Stoppcodon enthält. Dadurch können Teile des Vektors 3'wärts vom Insert in das Transcriptat übernommen werden, die für eine tag-Markierung kodieren.
d) gemeinsame PCR Amplifikation der beiden so erhaltenen Allelabschnitte als eine ununterbrochene Sequenz mittels zweier geeigneter Primer, insbesondere unter Verwendung des Anfangs-Primers aus Verfahrensschritt b, vorzugsweise AE1S, und des End-Primers aus Verfahrensschritt c, vorzugsweise AE8AS_WOS. e) Klonierung der amplifizierten ununterbrochenen Sequenz in einen Klonierungsvektor, der auch als Expressionsvektor geeignet ist z. B. pcDNA3.1, so dass ein Vektor-Insert Konstrukt entsteht, wie z.B. HLADE5pcDNA3.1. Alternativ kann auch ein einfacher Klonierungsvektor verwendet werden und das klonierte Insert später in einen beliebigen Expressionsvektor umkloniert werden. Das klonierte PCR Produkt enthält kein Stoppcodon, so dass auch Teile des Vektors 3'wärts vom Insert, in das Transcriptat übernommen werden können. Dadurch können Sequenzen, die für eine tag-Markierung kodieren und aus dem Vektor stammen, mit übernommen werden. Alternativ könnte der tag, der beliebig gewählt werden kann, auch im Primer AE8AS enthalten sein. In diesem Fall wird der Primer mit einem Stoppcodon 3'wärts vom tag versehen.
f) Expansion des so erhaltenen Plasmids in geeigneten Prokaryonten, vorzugsweise in kompetenten E. coli, und anschließende kontoaminationsfreie Aufreinigung des Plasmids z. B. mit Endofree Plasmid-Aufreinigungskit, so dass transfektierbare Plasmide gewonnen werden.
g) Transfektion von eukaryonten Zellen oder Zelllinien, z. B. K562 oder C1 R Zellen, mit dem Plasmid. Die Transfektion kann mit einer beliebigen Methode, z. B. Elektroporation, Lipofektion oder Calciumchlorid-Transfektion erfolgen. Die eukaryonten Zellen können beliebige Zellen sein, die natürlicherweise MHC Moleküle bilden können. Es können auch Zellen verwendet werden, die z.B. wegen fehlender Expression der zweiten Kette des MHC Moleküls bzw. der β2-Microglobulin bei HLA Klasse I, keine MHC Moleküle bilden können. In diesem Fall kann die fehlende Kette in einem weiteren Plasmid oder im gleichen Plasmid wie das beschriebene Insert co-transfiziert werden. Bei MHC Klasse II muss die zweite Kette, die α-Kette bei HLA Klasse II, co-transfiziert werden, da sie ebenfalls in der Membran verankert ist. Die co-transfizierte Kette kann auf die gleiche Weise wie das beschriebene Insert hergestellt werden oder durch Trunkierung der zweiten Kette, so dass nur der extrazelluläre Teil dieser Kette gebildet wird, erfolgen. Bei einer Transduktion wird homolog vorgegangen. Die transfizierten oder transduzierten Zellen bilden dann die rekombinanten MHC Moleküle, die in das umgebende Medium sezerniert werden, da den rekombinanten MHC Klasse I Molekülen der normalerweise durch Exon 5 kodierte transmembrane Abschnitt und den MHC Klasse II Molekülen der normalerweise durch Exon 4 kodierte transmembrane Abschnitt fehlt. Die rekombinanten MHC Moleküle können in einem ELISA nachgewiesen werden. Dazu können monoklonale Antikörper gegen MHC, z. B. w6/32 gegen HLA Klasse I, oder gegen den tag des Proteins, z. B. Anti-His oder Anti-V5 verwendet werden.
h) Die gentechnisch veränderten Zellen oder Zelllinien können in Zellkulturflaschen und anderen Zellkulturtechniken kultiviert und expandiert werden. Aus dem Überstand können die sezernierten, rekombinanten MHC Moleküle kontinuierlich geerntet werden. Alternativ können die produzierten Zelllinien auch in Hohlfaser-Bioreaktoren, z. B. der Firma Biovest (BioVest International, Inc., 8500 Evergreen Blvd. NW, Minneapolis, MN 55433, USA), kultiviert werden und das Medium kontinuierlich geerntet werden.
i) Aberntung der von den Zellen sezernierten, rekombinanten MHC-Moleküle.
j) Die rekombinanten Moleküle können gelchromatographisch und affinitätschromatorgraphisch aus dem geernteten Medium isoliert und gereinigt werden. Eine einfache Methode zur affinitätschromatographischen Aufreinigung besteht in der Anwendung eines monoklonalen Antikörpers gegen die tag-Sequenzen der rekombinanten Moleküle (z. B. antis-His, anti-V5).

Die beiden folgenden Tabellen zeigen jeweils in Tabelle 1 die genomische Organisation von HLA Klasse I Genen und in Tabelle 2 die genomische Organisation von HLA Klasse II Genen. Hierbei sind jeweils die nicht kodierenden Introns und die kodierenden Exons unter Angabe der Länge der Basenpaare bp und der korrespondierenden Aminosäureposition gezeigt.

**Tabelle 1 HLA Klasse I**

| **Transkribierte Region (mRNA)** | **Größe (bp)** | **Nichtkodierende Region** | **Größe (bp)** | **Aminosäure Position** |
|---|---|---|---|---|
| **5'UT** | 23 | **Promotor** | 200 | |
| **Exon** 1 | 73 | **Intron 1** | 130 | -24 - -1 |
| **Exon 2** | 270 | **Intron 2** | 241 | 1 - 90 |
| **Exon 3** | 276 | **Intron 3** | 601 | 91 - 182 |
| **Exon 4** | 276 | **Intron 4** | 97 | 183 - 274 |
| **Exon 5** | 117 | **Intron 5** | 438 | 275 - 313 |
| **Exon 6** | 33 | **Intron 6** | 140 | 314 - 324 |
| **Exon 7** | 48 | **Intron 7** | 169 | 325 - 340 |
| **Exon 8** | 5 | | | 341 |
| **3'UT** | 405 | | | |
| **Gesamtgröße** | 1,5 kb | | 2,0 kb | 341 AS |
| **Gesamtgröße des** | 3,5 kb | | | |
| **Gens** | | | | |
| **Gesamtgröße desProteins** | | | | 341 AS |

**Tabelle 2 HLA Klasse II**

| **Transkribierte Region (mRNA)** | **Größe (bp)** | **Nichtkodierende Region** | **Größe (bp)** | **Aminosäure Position** |
|---|---|---|---|---|
| **5'UT** | 62 | **Promotor** | >200 | |
| **Exon 1** | 100 | **Intron 1** | 8500 | -29 - +4 |
| **Exon 2** | 270 | **Intron 2** | 2250-2740 | 5 - 94 |
| **Exon 3** | 282 | **Intron 3** | 700 | 95 - 188 |
| **Exon 4** | 111 | **Intron 4** | 470 | 189 - 225 |
| **Exon 5** | 24 | **Intron 5** | 300-840 | 226 - 233 |
| **Exon 6** | 14 | | | 234 - 238 |
| **3'UT** | >365 | | | |
| **Gesamtgröße** | 1,2 kb | | 12,4-13,5 kb | 238 AA |
| **Gesamtgröße des Gens** | 13.6 - 14.7 kb | | | |
| **Gesamtgröße des Proteins** | | | | 238 AA |

Die Erfindung wird in einer bevorzugten Ausführungsform unter Bezugnahme auf eine Zeichnung beispielhaft beschrieben, wobei weitere vorteilhafte Einzelheiten den Figuren der Zeichnung zu entnehmen sind.

Die Figuren der Zeichnung zeigen im Einzelnen:
- Figur 1:: Eine schematische Darstellung der genomischen Organisation von HLA Klasse I Genen;
- Figur 2:: eine schematische Darstellung der genomischen Organisation wie in Figur 2, aber von HLA Klasse II Genen und
- Figur 3:: eine schematische Darstellung der PCR Strategie am Beispiel von H LA-A.

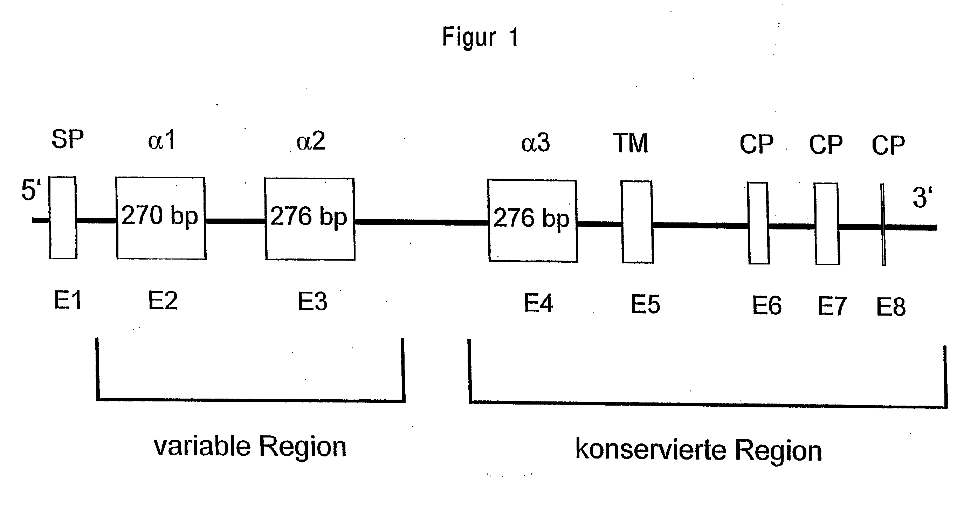

Figur 1 zeigt eine schematische Darstellung der genomischen Organisation von HLA Klasse I Genen. Hierbei ist die Nucleotidsequenz des Gens links von 5' nach rechts Richtung 3' dargestellt. Die Exons sind als Kästchen E1 bis E8 gezeigt, wobei der N-Terminus mit E1 beginnt und der C-Terminus an E8 endet. E1 ist das Signalpeptid SP, welches nach der intrazellulären Synthese des Proteins für den Transport über die Membran verantwortlich ist und E2 und E3 kodieren die α1 und α2 Domänen. Diese sind genetisch variabel und deshalb für die Bindung des endogenen Peptids verantwortlich. Die konservierte Region wird von den Exons E4 bis E8 gebildet. E 4 kodiert die α3 Domäne, E5 den Membranteil TM und die Exons E6 bis E8 CP den cytosolischen Schwanz.

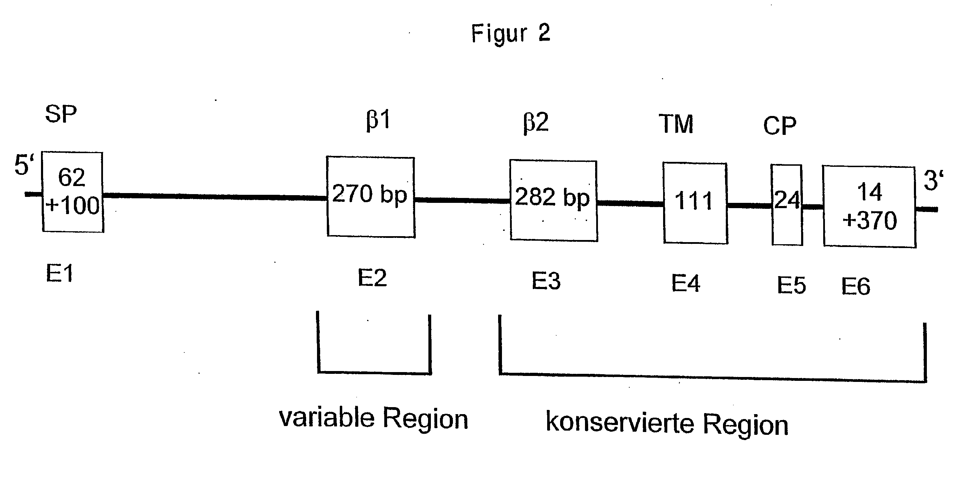

Figur 2 zeigt eine schematische Darstellung der genomischen Organisation wie in Figur 2, aber von HLA Klasse II Genen.

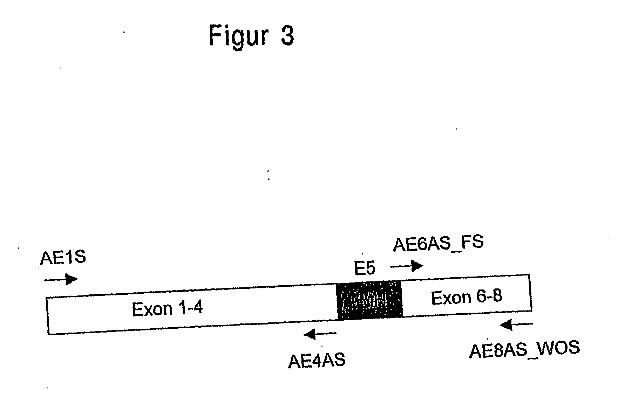

Figur 3 zeigt eine schematische Darstellung der PCR Strategie am Beispiel von HLA-A. Es wird somit das wesentliche Prinzip der Erfindung dargestellt. Die Figur zeigt die Gensequenz wie in Figur 1, aber ohne Introns. Zunächst werden mittels PCR mit dem ersten Anfangs-Primer AE1 S und dem ersten End-Primer AE4AS die kodierende Sequenz von Exon 1 bis Exon 4 amplifiziert. Dann wird vom gleichen Allel Exon 6 bis Exon 8 mit dem zweiten Anfangs-Primer AE6S_FS und dem zweiten End-Primer AE8AS_WOS amplifiziert. AE6S_FS enthält dabei eine 5' Sequenz, die zum 3' Ende von Exon 4 komplementär ist, so dass über diese Sequenz eine Fusionssequenz hergestellt werden kann. Die beiden so erhaltenen Sequenzen als eine ununterbrochene Sequenz werden zusammen mit AE1S als Anfangsprimer, und AE8AS_WOS als End-Primer amplifiziert. Es entsteht dadurch wieder die ursprüngliche ununterbrochene Sequenz, der allerdings das Exon 5 fehlt. Mit der Sequenz kann dann das nahezu native Protein ohne Membranteil in löslicher Form hergestellt werden.

Auf diese Weise ist ein lösliches rekombinante MHC Protein vorgeschlagen, das nicht trunkiert ist, und so auf überraschende Weise einen unveränderten C-Terminus aufweist.

## Patentansprüche

1. Rekombinantes, aufgereinigtes MHC-Protein, welches im Wesentlichen die gleiche Konformation, funktionelle Aktivität sowie die Bindungseigenschaften für spezifische Antikörper, Peptide, T-Zellrezeptoren und NK-Zellen und Antigene wie das native MHC-Protein aufweist, **dadurch gekennzeichnet, dass** das MHC-Protein keine transmembrane Domäne oder eine transmembrane Domäne aufweist, welche hinsichtlich ihrer Aminosäuresequenz derart modifiziert ist, dass das MHC-Protein löslich wird, und dass das MHC-Protein eine MHC-Protein intrazelluläre Domäne aufweist.

2. Rekombinantes MHC-Protein nach Anspruch 1, **dadurch gekennzeichnet, dass** es die Glykolisierung des nativen MHC-Proteins aufweist.

3. Rekombinantes MHC-Protein nach Anspruch 1 oder 2, **dadurch gekennzeichnet, dass** es mittels Affinitätschromatographie und/oder Gelchromatographie aufgereinigt ist.

4. Rekombinantes MHC-Protein nach einem der Ansprüche 1 bis 3, **dadurch gekennzeichnet, dass** es ein HLA-Protein der Klasse I oder der Klasse II ist.

5. Rekombinantes MHC-Protein nach einem der vorhergehenden Ansprüche, **dadurch gekennzeichnet, dass** es an seiner Peptid-bindenden Region ein dort gebundenes endogenes Peptid aufweist.

6. Rekombinantes MHC-Protein nach einem der vorhergehenden Ansprüche, **dadurch gekennzeichnet, dass** es im Wesentlichen nach folgendem Verfahren hergestellt ist:
a) Aufreinigung eines MHC Allels bestehend aus gDNA oder cDNA oder RNA,
b) PCR (Polymerase Chain Reaction) Amplifikation des MHC Allels von Exon 1 bis Exon 4 mit zwei geeigneten Primern, nämlich einen Anfangs-Primer und einen End-Primer,
c) PCR Amplifikation eines MHC Allels, nämlich des Allels des ersten oder zweiten Verfahrensschrittes, von Exon 6 bis Exon 7 oder Exon 8 mit zwei geeigneten Primern, nämlich einen Anfangs-Primer und einen End-Primer, wobei der Anfangs-Primer eine 5' Sequenz, die zum 3' Ende von Exon 4 komplementär ist, enthält, und wobei der End-Primer kein Stoppcodon enthält,
d) gemeinsame PCR Amplifikation der beiden so erhaltenen Allelabschnitte als eine ununterbrochene Sequenz mittels zweier geeigneter Primer, insbesondere unter Verwendung des Anfangs-Primers aus Verfahrensschritt b und des End-Primers aus Verfahrensschritt c,
e) Klonierung der amplifizierten ununterbrochenen Sequenz in einen Klonierungsvektor, der auch als Expressionsvektor geeignet ist, oder alternativ Umklonierung des Klonierungsvektors in einen Expressionsvektor,
f) Expansion des so erhaltenen Plasmids in geeigneten Prokaryonten, vorzugsweise E. coli, und anschließende Aufreinigung des Plasmids,
g) Transfektion oder Transduktion von eukaryonten Zellen oder Zelllinien mit dem so gewonnenen Plasmid,
h) Kultivierung und Expansion der Zellen oder Zelllinien mit geeigneten Methoden,
i) Aberntung der von den Zellen sezernierten, rekombinanten MHC-Moleküle und
j) Aufreinigung der Proteine mittels Affinitätschromatographie und/oder Gelchromatographie.

7. Verfahren zur Herstellung eines löslichen rekombinanten Proteins bestehend aus mindestens einer membranständigen und/oder transmembranen Domäne und einer oder mehrerer Domänen außerhalb der Membran, wobei ein das Protein kodierendes Gen jeweils für die Aminosäuresequenz jeder der Domänen kodierende Exons aufweist, **dadurch gekennzeichnet, dass** mittels PCR durch Wahl geeigneter Primer das oder die in einer Richtung der Sequenz benachbarten Exons amplifiziert werden, welche außerhalb der Membran befindliche Domänen kodieren und sich in genannter Richtung vor dem oder den Exons befinden, welches oder welche die Membran-Domäne kodiert, dann mittels PCR durch Wahl geeigneter Primer die in einer Richtung der Sequenz benachbarten Exons amplifiziert werden, welche außerhalb der Membran befindliche Domänen kodieren und sich in genannter Richtung hinter dem oder den Exons befinden, welches oder welche die Membran-Domäne kodiert, die so amplifizierten Sequenzen mittels PCR und geeigneter Primer, die eine überlappende Sequenz aufweisen, als ununterbrochene Sequenz amplifiziert werden, die ununterbrochene Sequenz , als Klonierungsvektor und/oder Expressionsvektor kloniert und/oder in Zellen expandiert wird, die so modifizierten das Protein ohne Membranteil produzierenden Zellen kultiviert werden und die exprimierten löslichen Proteine abgeernet und mittels üblicher oder sonstiger geeigneter Verfahren aufgereinigt werden.

## Claims

1. Recombinant, purified MHC protein which has basically the same conformation, functional activity, and binding properties for specific antibodies, peptides, T-cell receptors and NK cells and antigens as the native MHC protein, **characterised in that** the MHC protein does not have any transmembrane domains or has one transmembrane domain which is modified with regard to its amino acid sequence in such a way that the MHC protein is soluble, and **in that** the MHC protein has an MHC protein intracellular domain.

2. Recombinant MHC protein according to claim 1, **characterised in that** it comprises the glycolisation of the native MHC protein.

3. Recombinant MHC protein according to either claim 1 or claim 2, **characterised in that** it is purified by means of affinity chromatography and/or gel chromatography.

4. Recombinant MHC protein according to any one of claims 1 to 3, **characterised in that** it is an HLA protein of class I or class II.

5. Recombinant MHC protein according to any one of the preceding claims, **characterised in that** it has an endogenous peptide bound to its peptide-binding region.

6. Recombinant MHC protein according to any one of the preceding claims, **characterised in that** it is produced basically in accordance with the following method:
a) purification of an MHC allele consisting of gDNA or cDNA or RNA,
b) PCR (polymerase chain reaction) amplification of the MHC allele from exon 1 to exon 4 with two suitable primers, namely a start primer and an end primer,
c) PCR amplification of an MHC allele, namely the allele of the first or second method step, from exon 6 to exon 7 or exon 8 with two suitable primers, namely a start primer and an end primer, the start primer containing a 5' sequence that is complementary to the 3' end of exon 4, and the end primer containing no stop codon,
d) joint PCR amplification of the two allele segments thus obtained as an uninterrupted sequence by means of two suitable primers, in particular with use of the start primer from method step b and the end primer from method step c,
e) cloning of the amplified uninterrupted sequence into a cloning vector which is also suitable as an expression vector, or alternatively, re-cloning of the cloning vector into an expression vector,
f) expansion of the plasmid thus obtained in suitable prokaryotes, preferably E. coli, and subsequent purification of the plasmid,
g) transfection or transduction of eukaryotic cells or cell lines with the plasmid thus obtained,
h) cultivation and expansion of the cells or cell lines using suitable methods,
i) harvesting of the recombinant MHC molecules secreted by the cells, and
j) purification of the proteins by means of affinity chromatography and/or gel chromatography.

7. Method for producing a soluble recombinant protein consisting of at least one membrane-bound and/or transmembrane domain and one or more domains outside the membrane, a gene which codes for the protein in each case having exons which code for the amino acid sequence of each of the domains, **characterised in that** the exon(s) adjacent in one direction of the sequence are amplified by means of PCR by selecting suitable primers, said exon(s) coding for domains situated outside the membrane and being situated in said direction ahead of the exon(s) that code(s) for the membrane domain, then the exons that are adjacent in one direction of the sequence are amplified by means of PCR by selecting suitable primers, said exons coding for domains situated outside the membrane and being situated in said direction behind the exon(s) which code for the membrane domain, the sequences thus amplified being amplified as an uninterrupted sequence by means of PCR and suitable primers which have an overlapping sequence, the uninterrupted sequence being cloned as a cloning vector and/or expression vector and/or expanded in cells, the cells thus modified which produce the protein without the membrane part being cultivated and the expressed soluble proteins being harvested and purified by means of conventional or other suitable methods.

## Revendications

1. Protéine MHC recombinante purifiée, qui présente, pour l'essentiel, la même conformation, la même activité fonctionnelle ainsi que les mêmes propriétés de liaison pour des anticorps, des peptides, des récepteurs des cellules T et des cellules NK et des antigènes spécifiques que la protéine MHC native, **caractérisé en ce que** la protéine MHC ne présente aucun domaine transmembranaire ou présente un domaine transmembranaire, qui est modifié pour ce qui est de sa séquence d'aminoacides de telle sorte que la protéine MHC devienne soluble et que la protéine MHC présente un domaine intracellulaire de protéine MHC.

2. Protéine MHC recombinante selon la revendication 1, **caractérisé en ce qu'**elle présente la glycolisation de la protéine MHC native.

3. Protéine MHC recombinante selon la revendication 1 ou 2, **caractérisé en ce qu'**elle est purifiée par chromatographie d'affinité et/ou par chromatographie sur gel.

4. Protéine MHC recombinante selon l'une quelconque des revendications 1 à 3, **caractérisé en ce qu'**elle est une protéine HLA de la classe I ou de la classe II.

5. Protéine MHC recombinante selon l'une quelconque des revendications précédentes, **caractérisé en ce qu'**elle présente sur sa zone de liaison de peptides un peptide endogène qui y est fixé.

6. Protéine MHC recombinante selon l'une quelconque des revendications précédentes, **caractérisé en ce qu'**elle est fabriquée pour l'essentiel selon le procédé suivant :
a) Purification d'un allèle MHC se composant d'ADNg ou ADN ou d'ARN,
b) Amplification PCR (Polymerase Chain Reaction) de l'allèle MHC de l'exon 1 à l'exon 4 avec deux amorces appropriées, à savoir une amorce d'initiation et une amorce de fin,
c) Amplification PCR d'un allèle MHC, à savoir l'allèle de la première ou de la deuxième étape de procédé, de l'exon 6 à l'exon 7 ou à l'exon 8 avec deux amorces appropriées, à savoir une amorce d'initiation et une amorce de fin, sachant que l'amorce d'initiation contient une séquence 5' qui est complémentaire à la terminaison 3' de l'exon 4, et que l'amorce de fin ne contient aucun codon-stop,
d) Amplification PCR commune des deux tronçons d'allèle ainsi obtenus en tant que séquence ininterrompue avec deux amorces appropriées, en particulier par utilisation de l'amorce d'initiation provenant de l'étape de procédé b et de l'amorce de fin provenant de l'étape de procédé c,
e) Clonage de la séquence ininterrompue amplifiée en un vecteur de clonage, qui est approprié également en tant que vecteur d'expression, ou, alternativement, conversion du vecteur de clonage en un vecteur d'expression,
f) Expansion du plasmide ainsi obtenu dans des procaryotes appropriés, de préférence E. coli, et purification subséquente du plasmide,
g) Transfection ou transduction de cellules ou des lignées cellulaires eucaryotes à l'aide du plasmide ainsi obtenu,
h) Culture et expansion des cellules ou des lignées cellulaires grâce à des méthodes appropriées,
i) Collecte des molécules MHC recombinantes sécrétées par les cellules et
j) Purification des protéines par chromatographie d'affinité et/ou chromatographie sur gel.

7. Procédé pour la fabrication d'une protéine recombinante soluble se composant d'au moins un domaine membranaire et/ou transmembranaire et d'un ou de plusieurs domaines à l'extérieur de la membrane, sachant qu'un gène codant la protéine présente des exons codant à chaque fois pour la séquence d'aminoacides de chacun des domaines, **caractérisé en ce que**, via PCR grâce au choix d'amorces appropriées, on amplifie l'exon ou les exons voisin(s) dans une direction de la séquence, qui code ou codent les domaines se trouvant à l'extérieur de la membrane et se trouve ou se trouvent dans la direction citée avant le ou les exons, qui code ou codent les domaines de membrane, **en ce qu'**ensuite, via PCR grâce au choix de amorces appropriés, on amplifie les exons voisins dans une direction de la séquence, qui codent les domaines se trouvant à l'extérieur de la membrane et se trouvent dans la direction citée derrière le ou les exons, qui code ou codent les domaines de membrane, **en ce que** l'on amplifie via PCR et grâce à des amorces appropriées en tant que séquence ininterrompue, les séquences ainsi amplifiées, qui présentent une séquence superposée, **en ce que** l'on clone la séquence interrompue en tant que vecteur de clonage et/ou vecteur d'expression et/ou l'on procède dans les cellules à l'expansion de cette dernière, **en ce que** l'on procède à la culture des cellules ainsi modifiées produisant la protéine sans portion de membrane et **en ce que** l'on collecte et purifie, à l'aide de procédés usuels ou autres appropriés, les protéines solubles exprimées.


### SEQUENCE LISTING

<110> Imusyn GmbH & Co. KG
<120> verfahren zur Herstellung löslicher MHC-Proteine
<130> 2123
<140> DE 10 2004 022 435.8
   <141> 2004-05-06
<160> 15
<170> PatentIn version 3.1
<210> 1
   <211> 18
   <212> DNA
   <213> Homo sapiens
<400> 1
   gagatggccg tcatggcg 18
<210> 2
   <211> 20
   <212> DNA
   <213> Homo sapiens
<400> 2
   ccatctcagg gtgaggggct 20
<210> 3
   <211> 40
   <212> DNA
   <213> Homo sapiens
<400> 3
   ccctcaccct gagatgggat agaaaaggag ggagctactc 40
<210> 4
   <211> 22
   <212> DNA
   <213> Homo sapiens
<400> 4 cactttacaa gctgtgagag ac 22
   <210> 5
   <211> 1098
   <212> DNA
   <213> Homo sapiens
<400> 5
   atggccgtca tggcgccccg aaccctcgtc ctgctactct cgggggccct ggccctgacc     60
   cagacctggg cgggctccca ctccatgagg tatttctaca cctccgtgtc ccggcccggc    120
   cgcggggagc cccgcttcat cgccgtgggc tacgtggacg acacgcagtt cgtgcggttc    180
   gacagcgacg ccgcgagcca gaggatggag ccgcgggcgc cgtggataga gcaggagggg    240
   ccggagtatt gggaccggaa cacacggaat gtgaaggccc agtcacagac tgaccgagtg    300
   gacctgggga ccctgcgcgg ctactacaac cagagcgagg acggttctca caccatccag    360
   aggatgtatg gctgcgacgt ggggccggac gggcgcttcc tccgcgggta ccagcaggac    420
   gcttacgacg gcaaggatta catcgccctg aacgaggacc tgcgctcttg gaccgcggcg    480
   gacatggcgg ctcagatcac ccagcgcaag tgggagacgg cccatgaggc ggagcagtgg    540
   agagcctacc tggagggccg gtgcgtggag tggctccgca gatacctgga gaacgggaag    600
   gagacgctgc agcgcacgga cgcccccaag acgcatatga ctcaccacgc tgtctctgac    660
   catgaggcca ccctgaggtg ctgggccctg agcttctacc ctgcggagat cacactgacc    720
   tggcagcggg atggggagga ccagacccag gacacggagc tcgtggagac caggcctgca    780
   ggggatggga ccttccagaa gtgggcgtct gtggtggtgc cttctggaca ggagcagaga    840
   tacacctgcc atgtgcagca tgagggtctg cccaagcccc tcaccctgag atgggagccg    900
   tcttcccagc ccaccatccc catcgtgggc atcattgctg gcctggttct ctttggagct    960
   gtgatcgctg gagctgtggt cgctgctgtg atgtggagga ggaagagctc agatagaaaa   1020
   ggagggagct actctcaggc tgcaagcagt gacagtgccc agggctctga tatgtctctc   1080
   acagcttgta aagtgtga                                                 1098
<210> 6
   <211> 365
   <212> PRT
   <213> Homo sapiens
<400> 6
<210> 7
   <211> 341
   <212> PRT
   <213> Homo sapiens
<400> 7
   Gly Ser His Ser Met Arg Tyr Phe Tyr Thr Ser Val Ser Arg Pro Gly
   1 5 10 15
   Arg Gly Glu Pro Arg Phe Ile Ala Val Gly Tyr Val Asp Asp Thr Gln
   20 25 30
   Phe Val Arg Phe Asp Ser Asp Ala Ala Ser Gln Arg Met Glu Pro Arg
   35 40 45
   Ala Pro Trp Ile Glu Gln Glu Gly Pro Glu Tyr Trp Asp Arg Asn Thr
   50 55 60
   Arg Asn Val Lys Ala Gln Ser Gln Thr Asp Arg Val Asp Leu Gly Thr
   65 70 75 80
   Leu Arg Gly Tyr Tyr Asn Gln Ser Glu Asp Gly Ser His Thr Ile Gln
                   85                  90                  95
   Arg Met Tyr Gly Cys Asp Val Gly Pro Asp Gly Arg Phe Leu Arg Gly
               100                 105                 110
   Tyr Gln Gln Asp Ala Tyr Asp Gly Lys Asp Tyr Ile Ala Leu Asn Glu
           115                 120                 125
   Asp Leu Arg Ser Trp Thr Ala Ala Asp Met Ala Ala Gln Ile Thr Gln
       130                 135                 140
   Arg Lys Trp Glu Thr Ala His Glu Ala Glu Gln Trp Arg Ala Tyr Leu
   145                 150                 155                 160
   Glu Gly Arg Cys Val Glu Trp Leu Arg Arg Tyr Leu Glu Asn Gly Lys
                   165                 170                 175
   Glu Thr Leu Gln Arg Thr Asp Ala Pro Lys Thr His Met Thr His His
               180                 185                 190
   Ala Val Ser Asp His Glu Ala Thr Leu Arg Cys Trp Ala Leu Ser Phe
           195                 200                 205
   Tyr Pro Ala Glu Ile Thr Leu Thr Trp Gln Arg Asp Gly Glu Asp Gln
       210                 215                 220
   Thr Gln Asp Thr Glu Leu Val Glu Thr Arg Pro Ala Gly Asp Gly Thr
   225                 230                 235                 240
   Phe Gln Lys Trp Ala Ser Val Val Val Pro Ser Gly Gln Glu Gln Arg
                   245                 250                 255
   Tyr Thr Cys His Val Gln His Glu Gly Leu Pro Lys Pro Leu Thr Leu
               260                 265                 270
   Arg Trp Glu Pro Ser Ser Gln Pro Thr Ile Pro Ile Val Gly Ile Ile
           275                 280                 285
   Ala Gly Leu Val Leu Phe Gly Ala Val Ile Ala Gly Ala Val Val Ala
       290                 295                 300
   Ala Val Met Trp Arg Arg Lys Ser Ser Asp Arg Lys Gly Gly Ser Tyr
   305                 310                 315                 320
   Ser Gln Ala Ala Ser Ser Asp Ser Ala Gln Gly Ser Asp Met Ser Leu
                   325                 330                 335
   Thr Ala Cys Lys Val
               340
<210> 8
   <211> 1116
   <212> DNA
   <213> Homo sapiens
<400> 8
   atggccgtca tggcgccccg aaccctcgtc ctgctactct cgggggccct ggccctgacc      60
   cagacctggg cgggctccca ctccatgagg tatttctaca cctccgtgtc ccggcccggc     120
   cgcggggagc cccgcttcat cgccgtgggc tacgtggacg acacgcagtt cgtgcggttc     180
   gacagcgacg ccgcgagcca gaggatggag ccgcgggcgc cgtggataga gcaggagggg     240
   ccggagtatt gggaccggaa cacacggaat gtgaaggccc agtcacagac tgaccgagtg     300
   gacctgggga ccctgcgcgg ctactacaac cagagcgagg acggttctca caccatccag     360
   aggatgtatg gctgcgacgt ggggccggac gggcgcttcc tccgcgggta ccagcaggac     420
   gcttacgacg gcaaggatta catcgccctg aacgaggacc tgcgctcttg gaccgcggcg     480
   gacatggcgg ctcagatcac ccagcgcaag tgggagacgg cccatgaggc ggagcagtgg     540
   agagcctacc tggagggccg gtgcgtggag tggctccgca gatacctgga gaacgggaag     600
   gagacgctgc agcgcacgga cgcccccaag acgcatatga ctcaccacgc tgtctctgac     660
   catgaggcca ccctgaggtg ctgggccctg agcttctacc ctgcggagat cacactgacc     720
   tggcagcggg atggggagga ccagacccag gacacggagc tcgtggagac caggcctgca     780
   ggggatggga ccttccagaa gtgggcgtct gtggtggtgc cttctggaca ggagcagaga     840
   tacacctgcc atgtgcagca tgagggtctg cccaagcccc tcaccctgag atgggataga     900
   aaaggaggga gctactctca ggctgcaagc agtgacagtg cccagggctc tgatatgtct     960
   ctcacagctt gtaaagtgaa gggcaattct gcagatatcc agcacagtgg cggccgctcg    1020
   agtctagagg gcccgcggtt cgaaggtaag cctatcccta accctctcct cggtctcgat    1080
   tctacgcgta ccggtcatca tcaccatcac cattga                              1116
<210> 9
   <211> 135
   <212> DNA
   <213> Homo sapiens
<400> 9
   aagggcaatt ctgcagatat ccagcacagt ggcggccgct cgagtctaga gggcccgcgg      60
   ttcgaaggta agcctatccc taaccctctc ctcggtctcg attctacgcg taccggtcat     120
   catcaccatc accat                                                      135
<210> 10
   <211> 117
   <212> DNA
   <213> Homo sapiens
<400> 10
   agccgtcttc ccagcccacc atccccatcg tgggcatcat tgctggcctg gttctctttg 60
   gagctgtgat cgctggagct gtggtcgctg ctgtgatgtg gaggaggaag agctcag 117
<210> 11
   <211> 117 <212> DNA
   <213> Homo sapiens
<400> 11
   gagccgtctt cccagcccac catccccatc gtgggcatca ttgctggcct ggttctcttt 60
   ggagctgtga tcgctggagc tgtggtcgct gctgtgatgt ggaggaggaa gagctca 117
<210> 12
   <211> 371
   <212> PRT
   <213> Homo sapiens
<400> 12
   Met Ala Val Met Ala Pro Arg Thr Leu Val Leu Leu Leu Ser Gly Ala
   1 5 10 15
   Leu Ala Leu Thr Gln Thr Trp Ala Gly Ser His Ser Met Arg Tyr Phe
   20 25 30
   Tyr Thr Ser Val Ser Arg Pro Gly Arg Gly Glu Pro Arg Phe Ile Ala
   35 40 45
   Val Gly Tyr Val Asp Asp Thr Gln Phe Val Arg Phe Asp Ser Asp Ala
   50 55 60
   Ala Ser Gln Arg Met Glu Pro Arg Ala Pro Trp Ile Glu Gln Glu Gly
   65 70 75 80
   Pro Glu Tyr Trp Asp Arg Asn Thr Arg Asn Val Lys Ala Gln Ser Gln
   85 90 95
   Thr Asp Arg Val Asp Leu Gly Thr Leu Arg Gly Tyr Tyr Asn Gln Ser
   100 105 110
   Glu Asp Gly Ser His Thr Ile Gln Arg Met Tyr Gly Cys Asp Val Gly
   115 120 125
   Pro Asp Gly Arg Phe Leu Arg Gly Tyr Gln Gln Asp Ala Tyr Asp Gly
   130 135 140
   Lys Asp Tyr Ile Ala Leu Asn Glu Asp Leu Arg Ser Trp Thr Ala Ala
   145 150 155 160
   Asp Met Ala Ala Gln Ile Thr Gln Arg Lys Trp Glu Thr Ala His Glu
   165 170 175
   Ala Glu Gln Trp Arg Ala Tyr Leu Glu Gly Arg Cys Val Glu Trp Leu
   180 185 190
   Arg Arg Tyr Leu Glu Asn Gly Lys Glu Thr Leu Gln Arg Thr Asp Ala
   195 200 205
   Pro Lys Thr His Met Thr His His Ala Val Ser Asp His Glu Ala Thr
   210 215 220
   Leu Arg Cys Trp Ala Leu Ser Phe Tyr Pro Ala Glu Ile Thr Leu Thr
   225 230 235 240
   Trp Gln Arg Asp Gly Glu Asp Gln Thr Gln Asp Thr Glu Leu Val Glu
   245 250 255
   Thr Arg Pro Ala Gly Asp Gly Thr Phe Gln Lys Trp Ala Ser Val Val
   260 265 270
   Val Pro Ser Gly Gln Glu Gln Arg Tyr Thr Cys His Val Gln His Glu
   275 280 285
   Gly Leu Pro Lys Pro Leu Thr Leu Arg Trp Asp Arg Lys Gly Gly Ser
   290 295 300
   Tyr Ser Gln Ala Ala Ser Ser Asp Ser Ala Gln Gly Ser Asp Met Ser
   305 310 315 320
   Leu Thr Ala Cys Lys Val Lys Gly Asn Ser Ala Asp Ile Gln His Ser
   325 330 335
   Gly Gly Arg Ser Ser Leu Glu Gly Pro Arg Phe Glu Gly Lys Pro Ile
   340 345 350
   Pro Asn Pro Leu Leu Gly Leu Asp Ser Thr Arg Thr Gly His His His
   355 360 365
   His His His
   370
<210> 13
   <211> 24
   <212> PRT
   <213> Homo sapiens
<400> 13
   Met Ala Val Met Ala Pro Arg Thr Leu Val Leu Leu Leu Ser Gly Ala
   1 5 10 15
   Leu Ala Leu Thr Gln Thr Trp Ala
   20
<210> 14
   <211> 45
   <212> PRT
   <213> Homo sapiens
<400> 14
   Lys Gly Asn Ser Ala Asp Ile Gln His Ser Gly Gly Arg Ser Ser Leu
   1 5 10 15
   Glu Gly Pro Arg Phe Glu Gly Lys Pro Ile Pro Asn Pro Leu Leu Gly
   20 25 30
   Leu Asp Ser Thr Arg Thr Gly His His His His His His
   35 40 45
<210> 15
   <211> 347
   <212> PRT
   <213> Homo sapiens
<400> 15
   Gly Ser His Ser Met Arg Tyr Phe Tyr Thr Ser Val Ser Arg Pro Gly
   1 5 10 15
   Arg Gly Glu Pro Arg Phe Ile Ala Val Gly Tyr Val Asp Asp Thr Gln
   20 25 30
   Phe Val Arg Phe Asp Ser Asp Ala Ala Ser Gln Arg Met Glu Pro Arg
   35 40 45
   Ala Pro Trp Ile Glu Gln Glu Gly Pro Glu Tyr Trp Asp Arg Asn Thr
   50 55 60
   Arg Asn Val Lys Ala Gln Ser Gln Thr Asp Arg Val Asp Leu Gly Thr
   65 70 75 80
   Leu Arg Gly Tyr Tyr Asn Gln Ser Glu Asp Gly Ser His Thr Ile Gln
   85 90 95
   Arg Met Tyr Gly Cys Asp Val Gly Pro Asp Gly Arg Phe Leu Arg Gly
   100 105 110
   Tyr Gln Gln Asp Ala Tyr Asp Gly Lys Asp Tyr Ile Ala Leu Asn Glu
   115 120 125
   Asp Leu Arg Ser Trp Thr Ala Ala Asp Met Ala Ala Gln Ile Thr Gln
   130 135 140
   Arg Lys Trp Glu Thr Ala His Glu Ala Glu Gln Trp Arg Ala Tyr Leu
   145 150 155 160
   Glu Gly Arg Cys Val Glu Trp Leu Arg Arg Tyr Leu Glu Asn Gly Lys
   165 170 175
   Glu Thr Leu Gln Arg Thr Asp Ala Pro Lys Thr His Met Thr His His
   180 185 190
   Ala Val Ser Asp His Glu Ala Thr Leu Arg Cys Trp Ala Leu Ser Phe
   195 200 205
   Tyr Pro Ala Glu Ile Thr Leu Thr Trp Gln Arg Asp Gly Glu Asp Gln
   210 215 220
   Thr Gln Asp Thr Glu Leu Val Glu Thr Arg Pro Ala Gly Asp Gly Thr
   225 230 235 240
   Phe Gln Lys Trp Ala Ser Val Val Val Pro Ser Gly Gln Glu Gln Arg
   245 250 255
   Tyr Thr Cys His Val Gln His Glu Gly Leu Pro Lys Pro Leu Thr Leu
   260 265 270
   Arg Trp Asp Arg Lys Gly Gly Ser Tyr Ser Gln Ala Ala Ser Ser Asp
   275 280 285
   Ser Ala Gln Gly Ser Asp Met Ser Leu Thr Ala Cys Lys Val Lys Gly
   290 295 300
   Asn Ser Ala Asp Ile Gln His Ser Gly Gly Arg Ser Ser Leu Glu Gly
   305                 310                 315                 320
   Pro Arg Phe Glu Gly Lys Pro Ile Pro Asn Pro Leu Leu Gly Leu Asp
                   325                 330                 335
   Ser Thr Arg Thr Gly His His His His His His
               340                 345